# EUROPÄISCHE PATENTSCHRIFT

(11) **EP 1 000 154 B1**
(45) Veröffentlichungstag und Bekanntmachung des Hinweises auf die Patenterteilung: **21.02.2007**
(21) Anmeldenummer: 98942592.1
(22) Anmeldetag: 22.07.1998
(51) Int. Cl.: C12N 15/12, C12N 15/85, C12N 15/10, C07K 14/505

(54) **IDENTIFIZIERUNG HUMANER ZELLINIEN ZUR PRODUKTION HUMANER PROTEINE DURCH ENDOGENE GENAKTIVIERUNG**
IDENTIFICATION OF HUMAN CELL LINES FOR THE PRODUCTION OF HUMAN PROTEINS BY ENDOGENOUS GENE ACTIVATION
IDENTIFICATION DE LIGNEES CELLULAIRES HUMAINES POUR LA PRODUCTION DE PROTEINES HUMAINES PAR ACTIVATION GENIQUE ENDOGENE

(30) Priorität: 23.07.1997 EP 97112640; 01.12.1997 EP 97121073; 10.07.1998 US 113692
(43) Veröffentlichungstag der Anmeldung: 17.05.2000
(73) Patentinhaber: Roche Diagnostics GmbH, 68305 Mannheim (DE)
(72) Erfinder: BRANDT, Michael, D-82393 Iffeldorf (DE); FRANZE, Reinhard, D-82377 Penzberg (DE); PESSARA, Ulrich, D-82362 Weilheim (DE)
(74) Vertreter: Weiss, Wolfgang
(86) Internationale Anmeldenummer: PCT/EP1998/004584
(87) Internationale Veröffentlichungsnummer: WO 1999/005267

(56) Entgegenhaltungen:
- EP-A- 0 232 034
- EP-A- 0 267 678
- EP-A- 0 411 678
- EP-A- 0 747 485
- WO-A-93/09222
- WO-A-94/12650
- WO-A-95/31560
- WO-A-96/29411

## Beschreibung

Die Erfindung betrifft Verfahren zur Auswahl humaner Zellen für die Herstellung humaner Proteine durch endogene Genaktivierung, um humane Proteine in wirtschaftlichen Ausbeuten und in einer Form zu produzieren, die zur Herstellung eines pharmazeutischen Präparats geeignet ist. Weiterhin betrifft die Erfindung ein Verfahren zur Herstellung humaner Proteine in einer auf diese Weise identifizierten Zellinie.

Die Herstellung humaner Proteine durch endogene Genaktivierung in einer humanen Zellinie ist bekannt. So beschreiben beispielsweise WO93/09222, WO94/12650 und WO95/31560 die Produktion von humanem Erythropoietin und anderen humanen Proteinen in humanen Zellinien durch endogene Genaktivierung.

In den genannten Dokumenten findet sich jedoch keinerlei Hinweis, daß bei der Auswahl der zur Herstellung humaner Proteine verwendeten Zellinien bestimmte Kriterien zu beachten sind. Es kann daher nicht sichergestellt werden, daß das gewünschte humane Protein in der zu dessen Produktion ausgewählten Zellinie in der gewünschten Ausbeute und Form sowie frei von Kontaminationen erhältlich ist. Dementsprechend werden in den oben genannten Dokumenten im Allgemeinen auch nur geringe Ausbeuten an humanen Proteinen erreicht.

Aufgabe der vorliegenden Erfindung war es, die Nachteile des Standes der Technik zu beseitigen und insbesondere Kriterien zur Auswahl humaner Ausgangszellinien bereitzustellen, die für eine endogene Aktivierung eines vorbestimmten Zielgens geeignet sind.

Diese Aufgabe wird gelöst durch ein Verfahren zur Auswahl humaner Zellinien für die Herstellung humaner Proteine durch Aktivierung eines in der Zellinie endogen vorliegenden Zielgens, dadurch gekennzeichnet, daß man
(a) eine humane Zellinie auf das Vorliegen folgender Merkmale testet:
   (i) ein Zielgen mit der gewünschten Nukleinsäuresequenz,
   (ii) mindestens 5 Populationsverdopplungen innerhalb von 14 Tagen in einer Suspensionskultur, und
   (iii) mindestens 5 Populationsverdopplungen innerhalb von 14 Tagen in einem serumfreien Kulturmedium, und
(b) eine die Merkmale (i), (ii) und (iii) erfüllende Zellinie als Ausgangszelllinie für die endogene Aktivierung des Zielgens verwendet.

Wenn man vor der Aufgabe steht, ein humanes Zielgen in einer humanen Zellinie durch Gentargeting zu aktivieren und eine Zelle zu gewinnen, die zur Produktion des Zielproteins in zufriedenstellender Ausbeute und in der gewünschten Form in der Lage ist, wird man erfindungsgemäß mehrere Zellinien auf das Vorliegen einiger Merkmale testen, die diese Zellinie erfüllen muß, um ein geeigneter Kandidat für die spätere großtechnische Produktion des Zielproteins zu sein. Vorzugsweise werden als Zellinien dabei immortalisierte Zellinien, insbesondere Tumorzellinien, getestet, da diese gegenüber nichtimmortalisierten Zellen bedeutende Vorteile hinsichtlich der Kultivierbarkeit aufweisen.

Gemäß Merkmal (i) wird die humane Zellinie darauf untersucht, daß das Zielgen, d.h. das durch endogene Genaktivierung zu aktivierende Gen wirklich die gewünschte Nukleinsäuresequenz, im Allgemeinen die Nukleinsäuresequenz des natürlichen Zielgens aufweist. Tumorzellinien und andere Zellinien in permanenter Kultur zeigen nämlich oftmals eine Reihe von Mutationen in ihrem Genom. Daher ist ein wichtiger Aspekt bei der Auswahl einer geeigneten Zellinie, ob die Zellen ein korrektes Gen für das gewünschte Produkt besitzen. Die Sequenzbestimmung kann durch Züchtung der Zellen auf übliche Weise und Sequenzierung des Zielgens erfolgen. Gegebenenfalls kann vor der Sequenzierung eine Amplifizierung des Zielgens durch PCR oder andere Amplifikationsmethoden erfolgen.

Ein weiteres wichtiges Merkmal für die Auswahl einer Zellinie ist die Kultivierbarkeit in Suspension. Suspensionszellen sind leichter zur fermentieren und die Fermentation läßt sich leichter an größere Dimensionen, z.B. in einem Großfermenter mit einem Volumen von beispielsweise 10 I bis 50.000 I adaptieren. Daher sollten die ausgewählten Zellen entweder Suspensionszellen sein oder sie sollten sich leicht an eine Suspensionskultur adaptieren lassen. Hierzu werden die Zellen für 14 Tage unter ständigem Rühren kultiviert. Zeigen die Zellen innerhalb dieses Zeitraums mindestens fünf Populationsverdopplungen, werden sie als für eine Suspensionskultur geeignet angesehen. Die Bestimmung der Anzahl von Populationsverdoppelungen kann durch periodische Bestimmungen der Zellzahl, z.B. durch Zellzählung oder durch Messung der optischen Dichte der Zellsuspension erfolgen.

Ein weiteres wichtiges Merkmal für die Auswahl humaner Zellen ist die Kultivierbarkeit in serumfreiem Medium. Da die Aufreinigung von Proteinen aus serumfreien Zellkulturen wesentlich einfacher ist und in serumfreier Kultur keine Gefahr der Kontamination mit tierischen Pathogenen, z.B. Viren gegeben ist, sollten die ausgewählten Zellen in serumfreier Kultur gezüchtet werden können. Dazu werden die Zellen für 14 Tage in einer Dichte von 1 bis 10 x 10⁵ Zellen pro ml in Kulturgefäßen mit serumfreiem Medium (z.B. RPMI 1640 mit ITS von Boehringer Mannheim) kultiviert. Wenn die Zellen während dieser Kultur mindestens 5 Populationsverdopplungen, die durch Zellzählung bestimmt werden können, zeigen, werden sie als für serumfreie Kultur geeignet angesehen.

Ein weiteres wichtiges und erfindungsgemäß bevorzugtes Merkmal ist die Generationszeit (iv). So sollten die ausgewählten Zellen in Medien wie etwa DMEM 10% fötalem Kälberserum bzw. RPMI 1640 mit 10% fötalem Kälberserum eine hohe Proliferation aufweisen, d.h. sie sollten innerhalb einer Woche in Kultur 16 bis 256 Populationsverdopplungen, vorzugsweise 64 bis 128 Populationsverdoppelungen aufweisen. Dazu werden die Zellen in einer Konzentration von 0,1 bis 10 x 10⁵ Zellen pro ml, vorzugsweise 0,5 bis 2 x 10⁵ Zellen pro ml in Kulturschalen ausgesät und die Zellzahl alle zwei bis drei Tage mit Hilfe einer Zellkammer nach bzw. ohne Trypsinisierung bestimmt. Zellen, die eine ausreichend kurze Generationszeit aufweisen, sind für die großtechnische Produktion humaner Proteine durch endogene Genaktivierung besonders geeignet.

Ein weiteres bevorzugtes Merkmal ist das Fehlen einer nachweisbaren endogenen Expression, d.h. Transkription und Translation, des Zielgens (v). Vorzugsweise werden für die endogene Genaktivierung solche Zellinien ausgewählt, die im wesentlichen keine endogene Expression des Zielgens aufweisen. Hierzu können die Zellen für 24 Stunden in einer Zelldichte von 0,01 bis 2 x 10⁶ Zellen/ml vorzugsweise 0,5 bis 1 x 10⁶ Zellen/ml Kulturmedium ausgesät werden. Nach einer vorbestimmten Zeitdauer, z.B. 24 Stunden, wird der Zellüberstand abgenommen, die Zellen werden verworfen und der Gehalt des Zielproteins wird im Zellüberstand mittels bekannter Testverfahren, z.B. ELISA, bestimmt. Bei EPO liegt die Nachweisgrenze beispielsweise bei 10 pg/EPO/ml. Zellen, die bei einer Aussaat von 10⁶ Zellen/ml weniger als 10 pg Protein synthetisieren, werden als Nichtproduzenten angesehen und sind besonders geeignet.

Noch ein weiteres wichtiges und bevorzugtes Merkmal ist die Polysomie des Zielgens in der auszuwählenden Zelle (vi). Das Vorliegen von mehr als zwei chromosomalen Kopien des Zielgens in der Zelle erhöht die Ausbeuten bei der homologen Rekombination signifikant. Für die Produktion von EPO, dessen Gen auf dem Chromosom 7 liegt, haben sich beispielsweise die Zellen Namalwa (Nadkarni et al., Cancer 23 (1969), 64-79) oder HeLa S3 (Puck et al., J. Exp. Med. 103 (1956), 273-284), die das Chromosom 7 dreifach besitzen, als besonders geeignet erwiesen. Weitere Beispiele von Zellinien, die das Chromosom 7 in erhöhter Kopienzahl enthalten, sind die Colonadenokarzinomzellinie SW-480 (ATCC CCL-228; Leibovitz et al., Cancer Res. 36 (1976), 4562-4567), die maligne Melanomzellinie SK-MEL-3 (ATCC HTB 69; Fogh und Tremp, in: Human Tumor Cells in vitro, pp 115-159, J., Fogh (ed.), Plenum Press, New York 1975), die Colonadenokarzinomzelle Colo-320 (ATCC CCL-220; Quinn et al., Cancer Res. 39 (1979), 4914-4924)), die Melanomzellinie MEL-HO (DSM ACC 62; Holzmann et al., Int. J. Cancer 41 (1988), 542-547) und die Nierenkarzinomzellinie A-498 (DSM ACC 55; Giard et al., J. Natl. Cancer Inst. 51 (1973), 1417-1423).

Die Überprüfung der Anzahl von Chromosomen im Genom einer Zellinie kann durch Verwendung von DNA-Sonden geführt werden, die spezifisch für das jeweilige Chromosom oder/und den Locus des Zielgens sind.

Noch ein weiteres bevozugtes Merkmal einer für eine endogene Genaktivierung verwendeten Ausgangszellinie ist eine korrekte Glykosilierung des gewünschten Zielproteins (vii). Man verwendet vorzugsweise eine humane Zellinie, die das Zielprotein mit einem vergleichbaren Glykosilierungsmuster, insbesondere hinsichtlich der Anzahl der Sialinsäurereste wie das natürlich vorkommende Zielprotein synthetisiert. Der Test auf das Vorliegen einer korrekten Glykosilierung erfolgt vorzugsweise dadurch, daß man die zu testende Zelle transient mit einem extrachromosomalen Vektor transfiziert, welcher das gewünschte Zielgen unter Kontrolle eines in der Zelle aktiven Promotors enthält. Nach transienter Expression des Zielgens wird der Zellüberstand oder/und der Zellaufschluß durch isoelektrische Fokussierung analysiert. Am Beispiel von EPO wird das Vorliegen einer korrekten Glykosilierung sehr deutlich. So besitzt nichtglykosiliertes EPO, z.B. rekombinantes EPO aus E.coli Zellen, bei in vitro Experimenten eine vergleichbare Aktivität wie glykosiliertes EPO. Bei in vivo Experimenten ist nichtglykosiliertes EPO jedoch wesentlich weniger wirksam. Zur Bestimmung, ob eine Ausgangszellinie zur Herstellung von EPO mit einer korrekten Glykosilierung in der Lage ist, kann ein Vergleich mit urinärem EPO, aber auch mit rekombinantem EPO aus CHO Zellen erfolgen, von dem bekannt ist, daß es eine im Menschen aktive Form der Glykosilierung aufweist, und von seiner Glykosilierung weitgehend identisch mit dem urinären EPO ist. Der Vergleich der Glykosilierung erfolgt vorzugsweise durch isoelektrische Fokussierung.

Noch ein weiteres bevorzugtes Merkmal zur Auswahl einer humanen Zellinie ist die Freiheit der untersuchten Zellinie von infektiösen Kontaminationen (vii), z.B. von infektiösen viralen Partikeln oder Mycoplasmen. Die Untersuchungen auf das Vorhandensein viraler Kontaminationen kann mittels Zellkultur, in vivo Analysen oder/und Nachweis spezifischer viraler Proteine erfolgen.

Die Erfindung betrifft außerdem ein Verfahren zur Herstellung humaner Proteine durch endogene Genaktivierung in einer humanen Zellinie, welches dadurch gekennzeichnet ist, daß man eine Zellinie verwendet, welche die oben angegebenen Merkmale (i), (ii) und (iii) sowie vorzugsweise weiterhin mindestens eines der Merkmale (iv), (v), (vi) und (vii) wie zuvor angegeben erfüllt.

Das erfindungsgemäße Verfahren wird insbesondere zur Herstellung humaner Faktoren verwendet, beispielsweise EPO, Thrombopoietin (TPO), Kolonie-stimulierenden Faktoren wie G-CSF oder GM-CSF, Proteine, die die Blutgerinnung beeinflussen wietPA, Interferone wie IFN-α, IFN-β oder IFN-γ, Interleukine wie IL-1 bis IL-18, Chemokine wie MIP, neurotrophe Faktoren wie NGF oder BDNF, Proteine, die das Knochenwachstum beeinflussen wie IFG-BPs, Hedgehog (Igel)-Proteine, Tumorwachstumsfaktoren wie TFG-β, Wachstumshormone wie HGH, ACTH, Enkephaline, Endorphine, Rezeptoren wie etwa Interleukin- oder Insulinrezeptoren in löslichen oder/und membranständigen Formen und andere proteinbindende Proteine. Besonders bevorzugt wird das Verfahren zu Herstellung von EPO eingesetzt.

Die endogene Genaktivierung selbst kann nach bekannten Methoden erfolgen und umfaßt vorzugsweise die Schritte:
(a) Bereitstellen von humanen Ausgangszellinien, die mindestens eine Kopie eines endogenen Zielgens mit der gewünschten Nukleinsäuresequenz enthalten und durch Überprüfung der erfindungsgemäßen Auswahlkriterien als geeignet für die Expression des Zielgens identifiziert wurden,
(b) Transfizieren der Zellen mit einem DNA Konstrukt umfassend:
   (i) zwei flankierende DNA-Sequenzen, die homolog zu Bereichen des Zielgenlocus sind, um eine homologe Rekombination zu erlauben,
   (ii) ein positives Selektionsmarkergen,
   (iii) gegebenenfalls ein negatives Selektionsmarkergen,
   (iv) gegebenenfalls ein Amplifikationsgen und
   (v) eine heterologe Expressionkontrollsequenz, die in der humanen Zelle aktiv ist,
(c) Kultivieren der transfizierten Zellen unter Bedingungen, bei denen eine Selektion auf das Vorhandensein des positiven Selektionsmarkergens und gegebenenfalls auf die Abwesenheit des negativen Selektionsmarkergens stattfindet,
(d) Analysieren der gemäß Schritt selektierbaren Zellen,
(e) Identifizieren der das gewünschte Zielprotein produzierenden Zellen und
(f) gegebenenfalls Amplifizieren des Zielgens in den selektierten Zellen.

Das zur Herstellung der das gewünschte humane Protein produzierenden Zelle verwendete DNA-Konstrukt enthält zwei flankierende, zu Bereichen des Zielgenlocus homologe DNA-Sequenzen. Die Auswahl geeigneter flankierender Sequenzen erfolgt beispielsweise gemäß den in WO90/11354 und WO91/09955 beschriebenen Methoden. Vorzugsweise haben die flankierenden Sequenzen jeweils eine Lange von mindestens 150 bp.

Besonders bevorzugt werden die homologen DNA-Sequenzen aus dem 5'-Bereich des Zielgens, z.B. 5'-untranslatierte Sequenzen, Signalsequenzkodierende Exons und in diesem Bereich lokalisierte Introns, z.B. Exon1 und Intron 1 ausgewählt.

Das positive Selektionsmarkergen kann ein beliebiges für eukaryontische Zellen geeignete Selektionsmarkergen sein, welches bei Expression zu einem selektierbaren Phänotyp führt, z.B. Antibiotikumresistenz, Auxotrophie etc. Ein besonders bevorzugtes positives Selektionsmarkergen ist das Neomycinphosphotransferasegen.

Gegebenenfalls kann auch ein negatives Selektionsmarkergen wie etwa das HSV-Tymidinkinasegen, durch dessen Expression Zellen in Gegenwart eines Selektionsmittels zerstört werden, vorhanden sein. Das negative Selektionsmarkergen ist außerhalb der beiden flankierenden homologen Sequenzbereiche angeordnet.

Wenn eine Amplifikation des in der humanen Zelle endogen aktivierten Zielgens erwünscht wird, erhält das DNA-Konstrukt ein Amplifikationsgen. Beispiele für geeignete Amplifikationsgene sind das Dihydrofolatreduktasegen, das Adenosindeaminasegen, das Ornithindecarboxylasegen etc. Ein besonders bevorzugtes Amplifikationsgen ist das Dihydrofolatreduktasegen, insbesondere ein für eine Dihydrofolatreduktase-Arginin-Mutante kodierendes Gen, das eine höhere Sensitivität für das selektive Agens (Methotrexat) besitzt als das Wildtypgen (Simonsen et al., Proc. Natl. Acad, Sci. USA 80 (1983), 2495).

Weiterhin enthält das zur endogenen Genaktivierung verwendete DNA-Konstrukt eine heterologe Expressionskontrollsequenz, die in einer humanen Zelle aktiv ist. Die Expressionskontrollsequenz umfaßt einen Promotor und vorzugsweise weitere expressionsverbessernde Sequenzen, z.B. einen Enhancer. Der Promotor kann ein regulierbarer oder konstitutiver Promotor sein. Vorzugsweise ist der Promotor ein starker viraler Promotor, z.B. ein SV40- oder ein CMV-Promotor. Besonders bevorzugt ist der CMV-Promotor/Enhancer.

Weiterhin betrifft die Erfindung die Verwendung der durch das zuvor beschriebene Verfahren identifizierten humanen Zellinien nach Aktivierung eines in der Zelle endogen vorliegenden Zielgens zur Gewinnung des vom aktivierten Zielgen kodierenden Polypeptids, vorzugsweise zur Gewinnung des Polypeptids in einem großtechnischen Verfahren, z.B. unter Verwendung eines Großfermenters.

Noch ein weiterer Gegenstand der Erfindung ist eine humane Zelle, die eine Kopie eines endogenen Gens in operativer Verknüpfung mit einer heterologen, in der humanen Zelle aktiven Expressionskontrollsequenz enthält und ohne vorherige Genamplifikation zur Produktion von mindestens 200 ng des vom endogenen Gen kodierten Polypeptids/10⁶ Zellen/24 h in der Lage ist. Vorzugsweise ist die erfindungsgemäße humane Zelle zur Produktion von 200 bis 3000 ng Polypeptid/10⁶ Zellen/24 h und am meisten bevorzugt zur Produktion von 1000 bis 3000 ng Polypeptid/10⁶ Zellen/24 h in der Lage.

Schließlich noch ein weiterer Gegenstand der vorliegenden Erfindung ist eine humane Zelle, die durch Genamplifikation aus der zuvor genannten Zelle erhältlich ist und mehrere Kopien eines endogenen Gens jeweils in operativer Verknüpfung mit einer heterologen, in der humanen Zelle aktiven Expressionskontrollsequenz enthält und zur Produktion von mindestens 1000 ng des vom endogenen Gen kodierten Polypeptids/10⁶ Zellen/24 h in der Lage ist. Besonders bevorzugt ist die durch Genamplifikation erhältliche humane Zellinie zur Produktion von 1000 bis 25000 ng Polypeptid/10⁶ Zellen/24 h in der Lage und am meisten bevorzugt zur Produktion von 5000 bis 25000 ng/Polypeptid/10⁶ Zellen/24 h.

Ein Beispiel für eine erfindungsgemäße Zelle ist der EPO-produzierende Klon "Aladin" der am 15.07.1997 gemäß den Vorschriften des Budapester Vertrags bei der Deutschen Sammlung von Mikroorganismen und Zellkulturen (DSMZ), Mascheroder Weg 1 b, 38124 Braunschweig, unter dem Aktenzeichen DSM ACC 2320 hinterlegt wurde.

Weiterhin wird die Erfindung durch die nachfolgenden Beispiele und Abbildungen und Sequenzprotokolle erläutert.

Es zeigen:
- Abbildung 1:: Eine schematische Darstellung der Amplifikation von Homologieregionen des EPO-Gens aus dem Bereich der 5'-untranslatierten Sequenzen, Exon 1 und Intron 1;
- Abbildung 2:: Eine schematische Darstellung eines Plasmids, welches EPO-Homologieregionen aus dem Bereich der 5'-untranslatierten Sequenzen, Exon 1 und Intron 1 enthält;
- Abbildung 3:: Eine schematische Darstellung einer Genaktivierungssequenz, die den Rous-Sarcomavirus-Promotor (RSV), das Neomycinphosphotransferasegen (NEO), die frühe Polyadenylierungsregion von SV40 (SVI pA), den frühen SV40 Promotor (SVI), das Dihydrofolatreduktasegen (DHFR) und den Cytomegalovirus Immediate-earlyPromotor und Enhancer (MCMV) enthält;
- Abbildung 4a:: Die Herstellung des EPO-Gentargetingvektors p 176;
- Abbildung 4b:: Die Herstellung der EPO-Gentargetingvektoren p179 und p 187;
- Abbildung 4c:: Die Herstellung des EPO-Gentargetingvektors p189;

- Abbildung 4d:: Die Herstellung des EPO-Gentargetingvektors p190;
- Abbildung 4e:: Die Herstellung des EPO-Gentargetingvektors p192;
- SEQ ID NO. 1 und NO. 2: Nukleotidsequenzen der zur Herstellung des PCR Produkts 1 (Abb. 1) verwendeten Primer;
- SEQ ID NO. 3 und NO. 4: Sequenzen der zur Herstellung des PCR Produkts 2 (Abb. 1) verwendeten Primer.

### Beispiele

### Beispiel 1 Selektionsmethoden

### 1 . Bestimmung der Generationszeit

Die getesteten Zellinien wurden in einer Konzentration von 0,1 - 5 x 10⁵ pro ml, vorzugsweise von 0,5 - 2 x 105 Zellen pro ml in Kulturschalen mit DMEM und 10% FKS bzw. RPMI 1640 und 10% FKS ausgesät und die Zellzahl bei Kultivierung in einem für die jeweiligen Zellen z.B. von ATCC, empfohlenen Medium und unter geeigneten Bedingungen alle zwei bis drei Tage mit einer Zählkammer, z.B. Neubauer, ohne bzw. mit Trypsinisierung bestimmt. Zellen, die innerhalb einer Woche Kultivierung 16 bis 256 Populationsverdopplungen, vorzugsweise 64 bis 128 Populationsverdopplungen aufweisen, wurden als positiv (+, + + oder + + +) bewertet.

### 1.2 Kultivierbarkeit in Suspension

Zur Bestimmung der Kultivierbarkeit in Suspension wurden die Zellen für 14 Tage unter ständigem Rühren in Medium (siehe 1.1 mit und ohne Zusatz von Serum, z.B. fötalem Kälberserum) in einem Bellco-Spinner mit entsprechendem Zubehör bei 37°C und 7% CO₂ kultiviert. Zellen, die während dieser Phase mindestens 5 Populationsverdoppelungen zeigten, wurden als für eine Suspensionskultur geeignet (+) bewertet.

### 1.3 Kultivierbarkeit in serumfreien Medium

Um die Kultivierbarkeit von Zellen in serumfreiem Medium zu bestimmen, wurden die Zellen für 14 Tage in einer Dichte von 1 - 10 x 10⁵ Zellen/ml in Kulturgefäßen in den z.B. von ATCC für die jeweiligen Zellen empfohlenen Grundmedium (d.h. ohne Serumzusatz) mit ITS (Boehringer Mannheim, Kat.Nr. 1074547) unter Bedingungen gemäß 1.1 kultiviert. Zellen, die während dieser Zeit mindestens 5 Populationsverdoppelungen (bestimmt durch Zellzählung) aufwiesen, wurden als für die serumfreie Kultur geeignet (+) bewertet.

### 1.4 Bestimmung der endogenen Expression des Zielgens

Um zu bestimmen, ob das Zielprotein in der ausgewählten Zellinie endogen produziert wird, wurden die Zellen für 24 Stunden in einer Zelldichte von 0,01 bis 2 x 10⁶ Zellen/ml vorzugsweise 0,5 bis 1 x 10⁶ Zellen/ml Kulturmedium ausgesät. 24 Stunden später wurde der Zellüberstand abgenommen, die Zellen verworfen und der Gehalt des Zellproteins im Zellüberstand nach bekannten Methoden bestimmt, z.B. durch einen für das jeweilige Protein spezifischen Immunoassay bestimmt.

Im Fall von EPO wurde der Gehalt mittels ELISA bestimmt. Hierzu wurden Streptavidin-beschichtete Mikrotiterplatten mit biotinylierten Anti-EPO-Antikörpern (Boehringer Mannheim) beschichtet und zur Blockierung unspezifischer Bindungen mit einer Protein (1% w/v) enthaltenden Lösung inkubiert. Dann wurden 0,1 ml Kulturuberstand zugegeben und über Nacht inkubiert. Nach Waschen wurde Peroxidase-konjugierter monoklonaler Anti-EPO-Antikörper (Boehringer Mannheim) für zwei Stunden zugegeben. Die Peroxidasereaktion wurde unter Verwendung von ABTS^{®} als Substrat in einem Perkin Elmer Photometer bei 405 nm durchgeführt.

Die Nachweisgrenze von EPO in diesem Test lag bei 10 pg EPO/ml. Zellen, die bei einer Aussaat von 10⁶ Zellen/ml weniger als 10 pg EPO/ml erzeugen, wurden als Nichtproduzenten und als geeignet (+) bewertet.

### 1 .5 Bestimmung der Anzahl von Kopien des Zielgens

Zur Überprüfung der Anzahl von Kopien des Zielgens in der Zellinie, wurde humane genomische DNA aus ca. 10⁸ Zellen isoliert und quantifiziert (Sambrook et al., Molecular Cloning, A Laboratory Manual (1989), Cold Spring Harbor Laboratory Press). Nach Spaltung der DNA mit Restriktionsenzymen, z. B. Agel und Ascl bzw. BamHI, HindIII und Sall wurden die DNA-Fragmente durch Agarosegelelektrophorese ihrer Größe nach aufgetrennt und schließlich auf eine Nylonmembran transferiert und immobilisiert.

Die immobilisierte DNA wurde mit einer Digoxigenin-markierten DNA-Sonde hybridisiert, die spezifisch für den Locus des Zielgens oder das Chromosom, auf dem sich das Zielgen befindet, war und unter stringenten Bedingungen gewaschen. Die spezifischen Hybridisierungssignale wurden mit Hilfe eines Chemilumineszenzverfahrens unter Einsatz strahlungsempfindlicher Filme detektiert.

### 1.6 Bestimmung der Nukleinsäureseguenz des Zielgens

Aus ca. 10⁷ Zellen wurde die genomische DNA unter Verwendung eines DNA Isolierungskits (z.B. QIAGEN Blood & Cell Culture DNA Kit) isoliert.

Ein Paar von PCR-Primern wurde zur Amplifikation des Zielgens herangezogen. Die Sequenzen der Primer waren dabei komplementär zu Sequenzen, die die kodierende Region des Zielgens flankieren. Dadurch konnte die gesamte kodierende Region des Zielgens amplifiziert werden.

Das PCR-Produkt wurde direkt der Sequenzanalyse zugeführt oder in einen Vektor einkloniert und anschließend sequenziert. Dabei wurden Sequenzierprimer benutzt, deren Sequenzen komplementär zu Sequenzen aus den Intronbereichen des Zielgens sind, so daß die Sequenzen der Exonbereiche des Zielgens vollständig erhalten werden können. Die Sequenzierung erfolgte an einem PE/ABI-Sequenzierautomaten unter Verwendung z.B. des 'Prism^{™} Ready Reaction Dye Terminator Cycle Sequenzing' Kits (PE/ABI, Forster City, U.S.A.). nach den Angaben des Herstellers.

### 1.7 Bestimmung des Glykosilierungsmusters

Zur Bestimmung des Glykosilierungsmusters von EPO wurden die zu testenden Zellinien mit dem Plasmid pEPO 227 transfiziert, das ein 4 kb HindIII/EcoRI-Fragment der humanen EPO-Gensequenz unter Kontrolle des SV40 Promotors enthält (Jacobs et al. Nature 313 (1985), 806; Lee-Huang et al. Gene 128 (1993), 272). Dabei wurden die Zellen in Gegenwart von Lipofektamin unter Verwendung eines kommerziell erhältlichen Reagenzienkits nach Angaben des Herstellers transfiziert. In dem 2 bis 5 Tage später gewonnenen Zellüberstand wurde der EPO Gehalt mittels ELISA bestimmt.

Der Zellüberstand wurde konzentriert und durch isoelektrische Fokussierung (Righetti P.G., in: Work T.S., Burdon R.H. (ed.), lsoelectric focusing: Theory, methodology and applications, Elsevier Biomedical Press, Amsterdam (1983)) mit bekannten EPO-Produkten verglichen. Humane Zellen, die ein vergleichbares Glykosilierungsmuster zu bekannten EPO-Produkten, z.B. urinärem EPO lieferten, wurden als geeignet (+) bewertet.

### 1.8 Bestimmung viraler Kontaminationen

### 1.8.1 Analysen mittels Zellkultur

Zur Bestimmung möglicher infektiöser viraler Kontaminationen der zu testenden humanen Zellinie, wurde ein Lysat der Zellen mit einer Detektorzellinie inkubiert, um zytopatische Effekte nachweisen zu können. Zusätzlich wurden Hämoadsorptionsanalysen durchgeführt.

Zur Herstellung des Lysats wurde eine Suspension von 10⁶ Zellen in 1 ml Puffer durch einen schnellen Einfrier-Auftauprozess lysiert. Der zelluläre Rückstand wurde durch Zentrifugation abgetrennt und der Überstand zu den Detektorzellinien gegeben. Als Detektorzellinien wurden HepG2 (ATCC HB-8065; Nature 282 (1979), 615-616), MRC-5 (ATCC-1587) und Vero (ATCC CCL-171; Jacobs, Nature 227 (1970), 168-170) Zellen verwendet. Als Positivkontrolle wurden Viren vom Typ Polio SV und Influenza herangezogen. Als Negativkontrollen wurden Detektorzellinien verwendet, die ohne Lysat kultiviert wurden. Zur Bestimmung zytopatischer Effekte wurden die Detektorzellinien in einem Zeitraum von mindestens 14 Tagen regelmäßig untersucht.

Für die Hämadsorptionsanalyse wurden Verozellen, die mit den Zellysaten bzw. mit den Kontrollen inkubiert wurden, nach 7 Tagen mit Erythrozyten aus Huhn, Schwein oder Mensch versetzt. Eine Anheftung der Erythrozyten an den Monolayer der kultivierten Zellen weist auf eine virale Kontamination der Kulturen hin.

### 1.8.2 In vivo Analyse

Lysate der zu untersuchenden Zellinien wurden wie in 1.8.1 angegeben hergestellt und in frisch geborene Mäuse intraperitonial oder intaraceribral in einer Menge von 0,1 ml gespritzt. Über einen Zeitraum von 14 Tagen wurden die Mäuse im Hinblick auf die Morbidität und die Mortalität beobachtet. 1.8.3 Spezifischer Nachweis viraler Proteine

Die Gegenwart spezifischer viraler Proteine, z.B. Epstein-Barr-Virus-Proteinen (nukleäres Protein oder Capsidantigen) wurde getestet, in dem zu immobilisierten Zellen der zu testenden Zellinie humanes Serum von EBVpositiven Bändern zugegeben wurde. Der Nachweis der Virusantigene erfolgte dann über Zugabe von Komplement und dem entsprechenden Anti-human-Komplement C3 Fluorescein-Konjugat (zum Nachweis des nukleären Antigens) bzw. über Anti-human-Globulinfluorescein (zum Nachweis des Capsidantigens).

### Beispiel 2 Selektionsergebnisse

Es wurden die humanen Zellinien HepG2, HT1080, Namalwa, Hela und HelaS3 nach den in Punkt 1 angegebenen Methoden getestet. Die Ergebnisse sind in der folgenden Tabelle 1 dargestellt.

**Tabelle 1**

| | | | | | |
|---|---|---|---|---|---|
| | HepG2 | HT1080 | Namalwa | Hela | Hela S3 |
| **Wachstumsverhalten** | | | | | |
| Verdopplungszeit | + | ++ | ++ | +++ | +++ |
| Suspensionskultur | - | + | + | +/- | + |
| Serumfrei möglich | +/- | + | + | + | + |
| **EPO-Produktion** | | | | | |
| endogen | + | - | - | - | - |
| transient/ml | 53 ng | 65 ng | 70 ng | 100 ng | 480 ng |
| **EPO-Glykosylierung (transient)** | - | + | + | + | + |
| **Test auf virale infektiöse Kontaminationen** | nicht getestet | frei | frei | nicht getestet | frei |

Aus Tabelle 1 ist ersichtlich, daß die Zellinien HT1080, Namalwa und Hela S3 als geeignet für die endogene Genaktivierung von EPO anzusehen sind. Besonders geeignet sind Namalwa und Hela S3.

### Beispiel 3 Klonierung von EPO-Homologieregionen

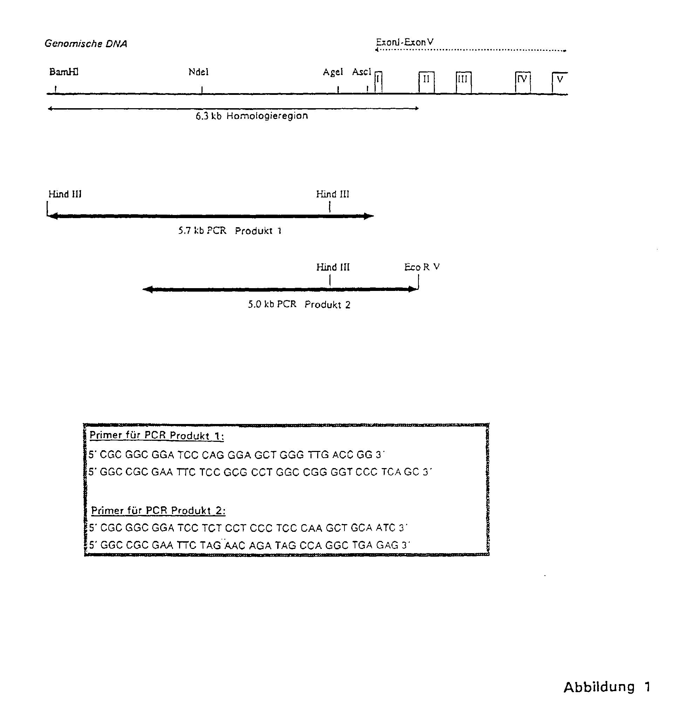

Homologieregionen des EPO-Gens wurden durch PCR unter Verwendung einer genomischen Placenta DNA (Boehringer Mannheim) amplifiziert. Dabei wurden aus einer 6,3 kB langen Homologieregion aus dem Bereich der 5'-untranslatierten Sequenzen des EPO-Gens, Exon 1 und Intron 1 zwei PCR-Produkte hergestellt (vgl. Figur 1). Die zur Herstellung des PCR-Produkts 1 verwendeten Primer hatten folgende Sequenzen: 5'-CGC GGC GGA TCC CAG GGA GCT GGG TTG ACC GG-3' (SEQ ID NO. 1) und 5'-GGC CGC GAA TTC TCC GCG CCT GGC CGG GGT CCC TCA GC-3' (SEQ ID NO. 2). Die zur Herstellung des PCR-Produkts 2 verwendeten Primer hatten folgende Sequenzen: 5'-CGC GGC GGA TCC TCT CCT CCC TCC CAA GCT GCA ATC-3' (SEQ ID NO. 3) und 5'-GGC CGC GAA TTC TAG AAC AGA TAG CCA GGC TGA GAG-3' (SEQ ID NO. 4).

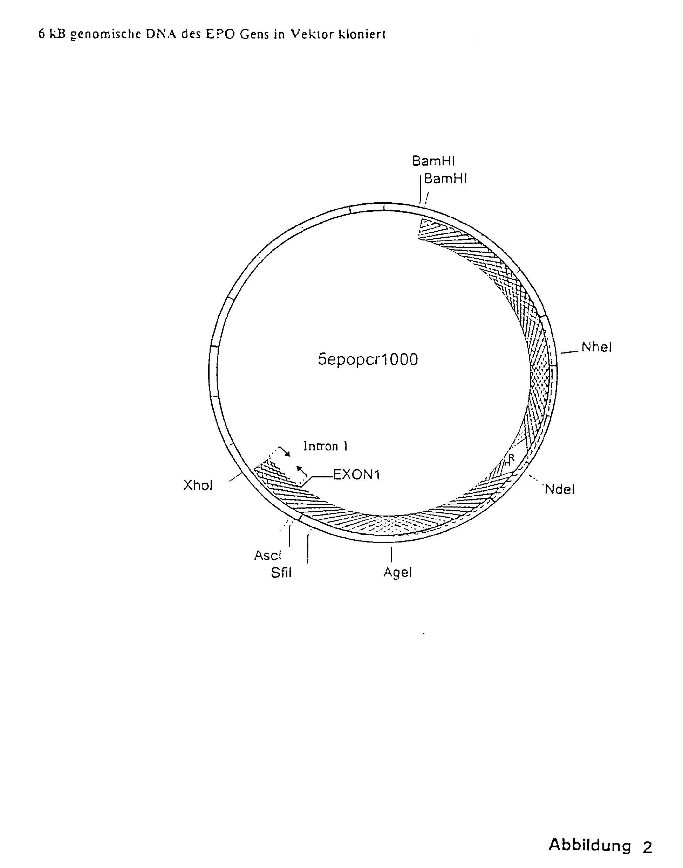

Aus den PCR-Produkten 1 und 2 wurden die gewünschten Segmente durch Restriktionsspaltung (PCR-Produkt 1: HindIII, PCR-Produkt 2: HindIII und Eco RV) herausgeschnitten und in den Vektor pCRII (Invitrogen), der mit HindIII und Eco RV gespalten war, kloniert. Der auf diese Weise erhaltene rekombinante Vektor wurde 5epopcr 1000 bezeichnet (vgl. Figur 2).

### Beispiel 4 Konstruktion von EPO Gen Targetingvektoren

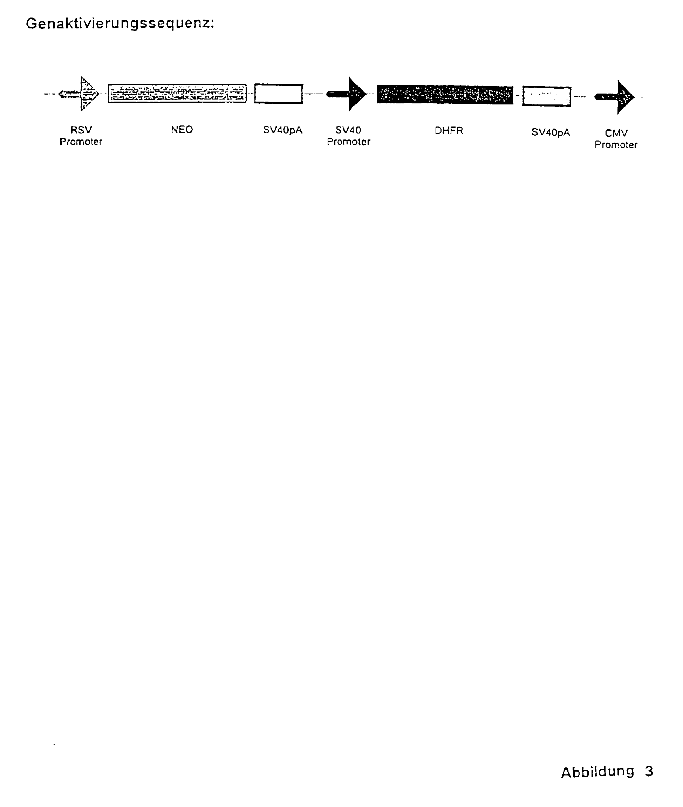

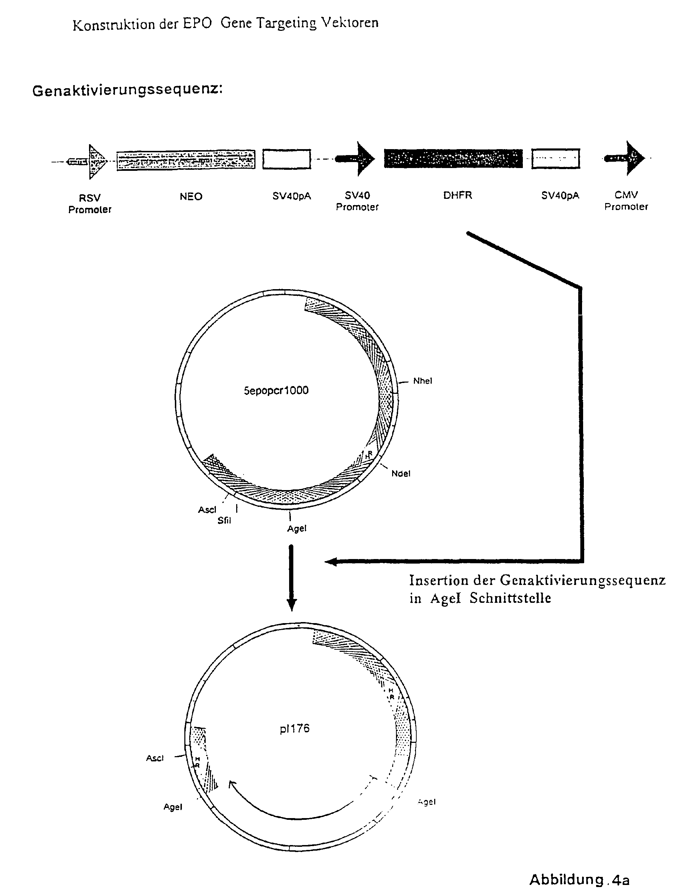

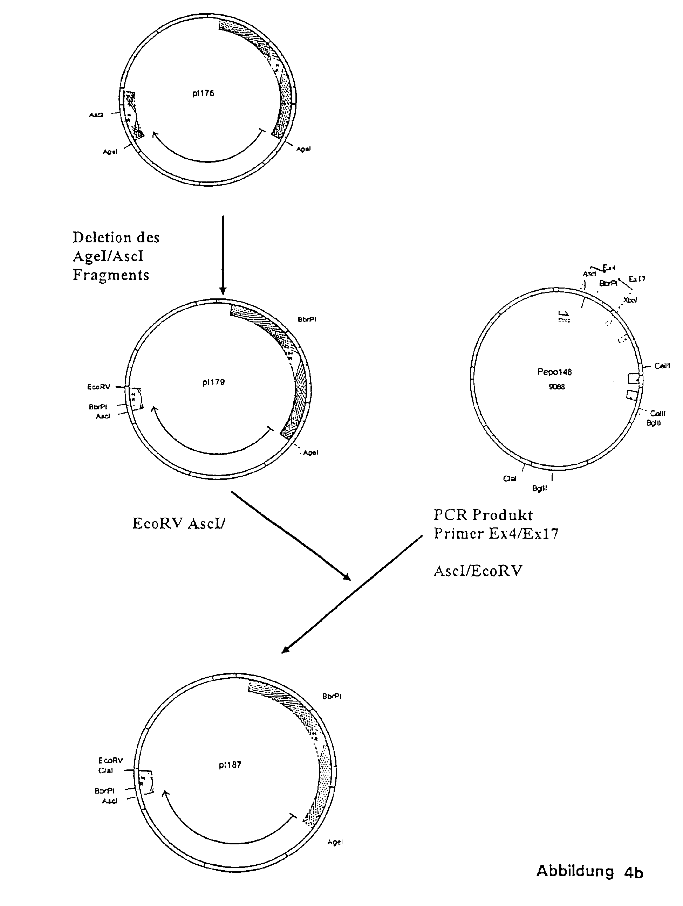

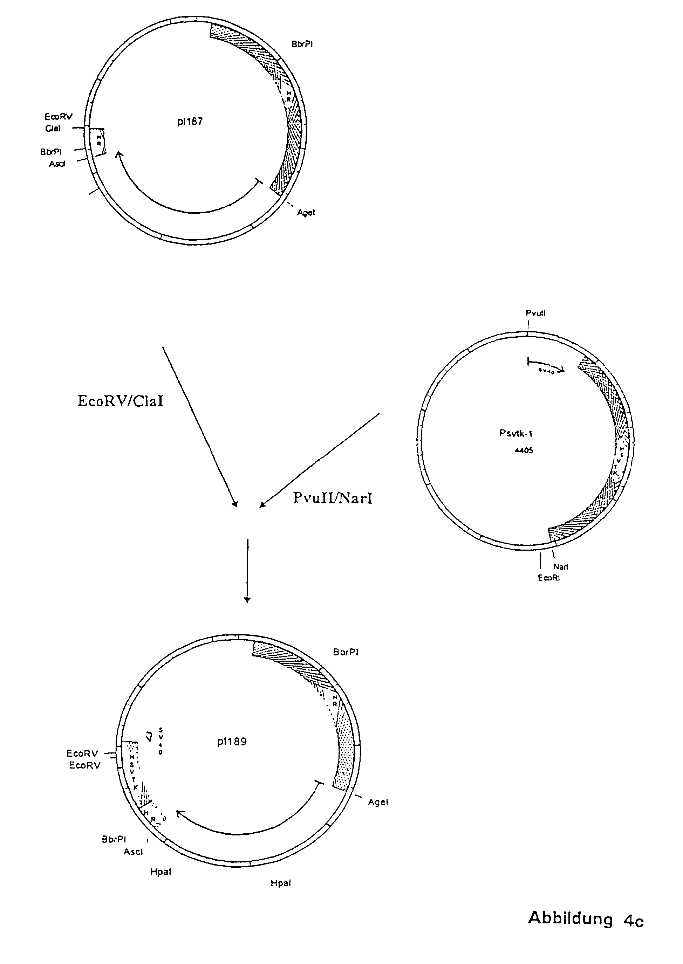

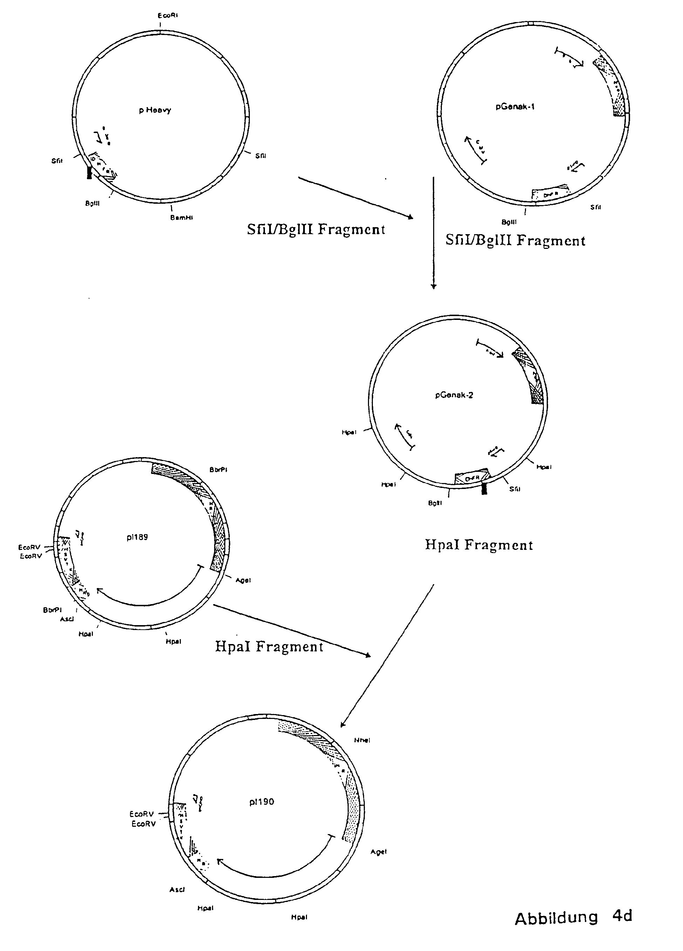

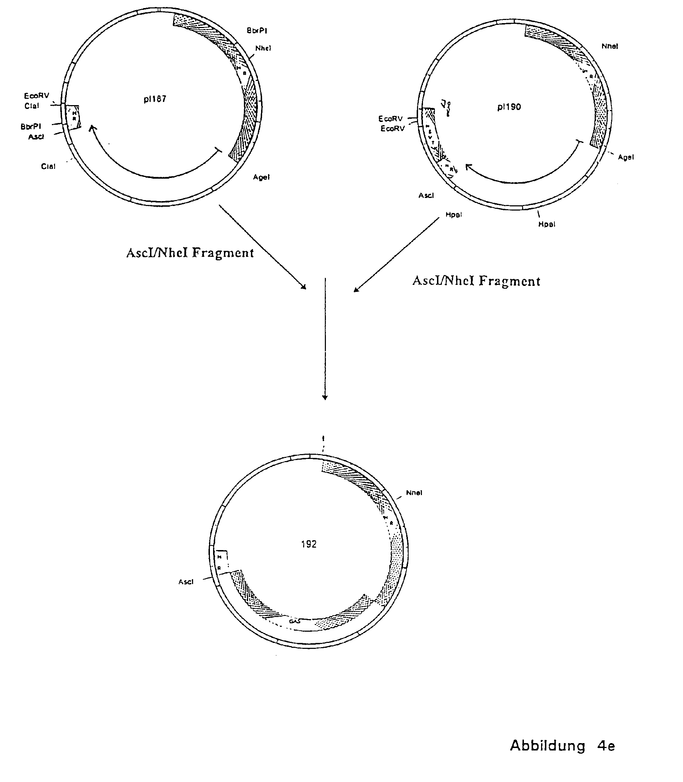

4.1 Eine Genaktivierungssequenz, die das NEO-Gen, das DHFR Gen und einen CMV Promotor/Enhancer enthält (vgl. Fig. 3) wurde in die Agel Stelle des die EPO Homologieregion enthaltenden Plasmids 5epocr1000 inseriert, wobei das Plasmid p176 erhalten wurde (vgl. F!G. 4a). Um den CMV Promotor möglichst nahe an die Translationsstartstelle des EPO Gens zu bringen, wurde ein 963 bp langes Segment zwischen den Restriktionsstellen Ascl und Agel (partielle Spaltung) deletiert, wobei das Plasmid p 179 erhalten wurde (Fig. 4b).
4.2 Um eine Optimierung der Expression zu erreichen, wurden Nukleotide in Exon 1, die für den Beginn der EPO-Leader-sequenz Met-Gly-Val-His kodieren, durch die synthetische Sequenz Met-Ser-Ala-His ersetzt. Diese Sequenz wurde durch Amplifikation einer genomischen EPO-DNA-Sequenz, z.B. des Plasmids pEPO148, das ein 3,5 kB BstEII/EcoRI-Fragment (inklusive der Exons 1-5) der humanen EPO Gensequenz unter Kontrolle des SV40 Promotors enthält (Jacobs et al., Nature 313 (1985), 806 und Lee-Huang et al., Gene 128 (1993), 227), als Matrize mit entsprechenden Primern erhalten. Dabei wurde das Plasmid p187 erhalten (Fig. 4b).
4.3 Das Plasmid p 189 wurde aus dem Plasmid p 187 durch Insertion des Herpes Simplex Virus-Thymidinkinasegens (HSV-TK), welches aus Psvtk-1 (Pvull/Narl Fragment) stammte, hergestellt (Fig. 4c). Das HSV-TK-Gen befindet sich unter Kontrolle des SV40 Promotors am 3'-Ende von Intron 1 (Eco RV/Clal) in entgegengesetzter Orientierung relativ zum CMV Promotor und sollte zur negativen Selektion auf eine homologe Rekombination dienen.
4.4 Zur Konstruktion von Plasmid p190 wurde ein SfiI/BglII Fragment von pHEAVY, einem Plasmid, das die cDNA einer bei Simonsen et al. (Proc. Natl. Acad. Sci. USA 80 (1983), 2495) beschriebene Arginin-Mutante von DHFR enthält, in das mit Sfil und BglII geschnittene Plasmid pGenak-1 subkloniert, das das NEO-Gen unter Kontrolle des RSV-Promotors und der späten SV40 Polyadenylierungsstelle als Terminator, das murine DHFR-Gen unter Kontrolle des frühen SV40 Promotors und der frühen SV40 Polyadenylierungsstelle als Terminator (Kaufmann et al., Mol. Cell. Biol. 2 (1982), 1304; Okayama et al., Mol. Cell. Biol. 3 (1983), 280 und Schimke, J. Biol. Chem. 263 (1988), 5989) und den CMV-Promotor (Boshart et al., Cell 41 (1995), 521) enthält. Anschließend wurde ein Hpal Fragment, welches die für die DHFR-Arginin-Mutante kodierende cDNA enthielt, in das mit Hpal geschnittene Plasmid p189 ligiert, wobei das Plasmid p190 erhalten wurde (Fig. 4d).
4.5 Um einen Transfektionsvektor ohne das HSV-TK Gen zu erhalten, wurde ein Ascl/Nhel Fragment des Plasmid p190, welches die Genaktivierungssequenz enthielt, in ein das Exon 1 enthaltendes Ascl/Nhel Fragment des Plasmids p187 ligiert. Das resultierende Plasmid wurde p192 genannt (Fig. 4e).

### Beispiel 5 Transfektion von Zellen

Es wurden verschiedene Zellinien auf die Produktion von EPO selektioniert und mit Targetingvektoren transfiziert.

### 5.1 Namalwazellen

Die Zellen wurden in T150 Gewebekulturflaschen gezüchtet und durch Elektroporation (1 x 10⁷ Zellen/800 µl Elektroporationspuffer 20 mM Hepes, 138 mM NaCl, 5 mM KCl, 0,7 mM Na₂HPO₄, 6 mM D-Glucose-Monohydrat pH 7,0, 10 µg linearisierte DNA, 960 µF, 260 V BioRad Gene Pulser) transfiziert. Nach der Elektroporation wurden die Zellen in RPMI 1640, 10% (v/v) fötalem Kälberserum (FCS), 2 mM L-Glutamin, 1 mM Natriumpyruvat in vierzig 96-Lochplatten gezüchtet. Nach zwei Tagen wurden die Zellen für 10 bis 20 Tage in 1 mg/ml G-418 enthaltendem Medium kultiviert. Der Überstand wurde in einem Festphasen-ELISA auf die Produktion von EPO getestet (siehe Beispiel 1.4). Die EPO produzierenden Klone wurden in 24-Lochplatten und T-25 Gewebekulturflaschen expandiert. Aliquots wurden eingefroren und die Zellen wurden durch FACS (Ventage, Becton Dickinson) subkloniert. Die Subklone wurden wiederholt auf EPO-Produktion getestet.

### 5.2 HT 1080 Zellen

Die Bedingungen waren wie für Namalwazellen beschrieben, außer daß die HT1080 Zellen in DMEM, 10% (v/v) FCS, 2 mM L-Glutamin, 1 mM Natriumpyruvat kultiviert wurden. Zur Transfektion durch Elektroporation wurden die Zellen durch Typsinisierung von den Wänden der Kulturgefäße abgelöst. Nach der Elektroporation wurden 1 x 10⁷ Zellen in DMEM, 10% (v/v) FCS, 2 mM L-Glutamin, 1 mM Natriumpyruvat in 5 96-Lochplatten kultiviert.

### 5.3 HeLa S3 Zellen

Die Bedingungen waren wie für Namalwalzellen beschrieben, außer daß die HeLaS3-Zellen in RPMI 1640, 10% (v/v) FCS, 2 mM L-Glutamin, 1 % (v/v) NEM nichtessentiellen Aminosäuren (Sigma), 1 mM Natriumpyruvat kultiviert wurden. Zur Transfektion durch Elektroporation wurden die Zellen durch Trypsinisierung von den Wänden der Kulturgefäße abgelöst. Die Bedingungen für die Elektroporation waren 960 µF/250 V. Nach der Elektroporation wurden die Zellen in RPMI 1640, 10% (v/v) FCS, 2 mM L-Glutamin, 1 % (v/v) NEM, 1 mM Natriumpyruvat in T75 Gewebekulturflaschen kultiviert. 24 Stunden nach Elektroporation wurden die Zellen trypsinisiert und für 10 bis 15 Tage in einem 600 µg/ml G-41 8 enthaltenden Medium in 10 96-Lochplatten kultiviert.

### Beispiel 6 EPO-Genamplifizierung

Zur Erhöhung der EPO Expression wurden die EPO produzierenden Klone in Gegenwart steigender Konzentrationen (100 pM-1000 nM) Methotrexat (MTX) kultiviert. Bei jeder MTX Konzentration wurden die Klone durch einen ELISA (siehe Beispiel 1.4) auf Produktion von EPO getestet. Starke Produzenten wurden durch limitierte Verdünnung subkloniert.

### Beispiel 7 Charakterisierung von EPO-produzierenden Zellinien

Drei verschiedene Zellinien (Namalwa, HeLa S3 und HT 1080) wurden zur EPO Genaktivierung ausgewählt. EPO-produzierende Klone wurden durch Transfektion mit den Plasmiden p179, p187, p189, p190 oder p192 erhalten (vgl. Beispiele 2 und 3).

Es wurden ca. 160.000 NEO-resistente Klone auf die EPO-Produktion getestet, von denen 12-15 EPO in signifikanter Ausbeute reproduzierbar in den Zellüberstand sekretierten.

Davon konnten überraschenderweise ohne Genamplifikation durch MTX insgesamt 7 EPO-Klone identifiziert werden, die EPO in ausreichenden Mengen für ein großtechnische Produktion erzeugten. Die EPO-Produktion dieser Klone lag im Bereich von 200 ng/ml bis mehr als 1000 ng/ml/10⁶ Zellen/24 h.

Nach Genamplifikation mit 500 nM MTX konnte die EPO-Produktion der identifizierten EPO-Klone auf bis zu mehr als 3000 ng/ml/10⁶ Zellen/24 h gesteigert werden. Eine weitere Erhöhung der MTX Konzentration auf 1000 nM führte zu einer Produktion von bis zu mehr als 7000 ng/ml/10⁶ Zellen/24 h.

Die erhaltenen Klone zeigten eine EPO Produktion auch unter serumfreien Kulturbedingungen und in Abwesenheit von MTX.

## Patentansprüche

1. Verfahren zur Auswahl humaner Zellinien für die Herstellung humaner Proteine durch endogene Aktivierung eines in der Zelle endogen vorliegenden Zielgens,
**dadurch gekennzeichnet,**
**daß** man
(a) eine humane Zellinie auf das Vorliegen folgender Merkmale testet:
(i) ein Zielgen mit der gewünschten Nukleinsäuresequenz,
(ii) mindestens 5 Populationsverdopplungen innerhalb von 14 Tagen in einer Suspensionskultur, und
(iii) mindestens 5 Populationsverdopplungen innerhalb von 14 Tagen in einem serumfreien Kulturmedium, und
(b) eine die Merkmale (i), (ii) und (iii) erfüllende Zellinie als Ausgangszellinie für die endogene Aktivierung des Zielgens verwendet.

2. Verfahren nach Anspruch 1,
**dadurch gekennzeichnet,**
**daß** man eine humane Zellinie verwendet, die (iv) eine Generationszeit von 16 bis 256 Populationsverdopplungen innerhalb einer Woche in einem Kulturmedium aufweist.

3. Verfahren nach Anspruch 2,
**dadurch gekennzeichnet,**
**daß** man eine humane Zellinie verwendet, die 64 bis 128 Populationsverdopplungen innerhalb einer Woche in einem Kulturmedium aufweist.

4. Verfahren nach einem der vorhergehenden Ansprüche,
**dadurch gekennzeichnet,**
**daß** man eine humane Zellinie verwendet, die (v) im wesentlichen keine endogene Expression des Zielgens aufweist.

5. Verfahren nach einem der vorhergehenden Ansprüche,
**dadurch gekennzeichnet,**
**daß** man eine humane Zellinie verwendet, die (vi) mehr als 2 chromosomale Kopien des Zielgens enthält.

6. Verfahren nach einem der vorhergehenden Ansprüche,
**dadurch gekennzeichnet,**
**daß** man eine humane Zellinie verwendet, die (vii) das Zellprotein mit einem vergleichbaren Glykosilierungsmuster wie das natürlich vorkommende Zielprotein synthetisiert.

7. Verfahren nach einem der vorhergehenden Ansprüche,
**dadurch gekennzeichnet,**
**daß** man eine humane Zelline verwendet, die (viii) frei von nachweisbaren infektiösen Kontaminationen ist.

8. Verfahren zur Herstellung humaner Proteine durch endogene Genaktivierung in einer humanen Zelle,
**dadurch gekennzeichnet,**
**dass** durch ein Verfahren nach einem der Ansprüche 1 bis 7 eine Zellinie ausgewählt wird, welche die Merkmale (i), (ii) und (iii) wie in Anspruch 1 definiert erfüllt, und die so ausgewählte Zellinie verwendet wird.

9. Verfahren nach Anspruch 8,
**dadurch gekennzeichnet,**
**daß** man eine Zellinie verwendet, die weiterhin mindestens eines der Merkmale (iv), (v), (vi), (vii) und (viii) wie in einem der Ansprüche 2, 4, 5, 6 und 7 definiert erfüllt.

10. Verfahren nach Anspruch 8 oder 9 zur Herstellung eines humanen Faktors ausgewählt aus EPO, TPO, koloniestimulierenden Faktoren, Proteinen, die die Blutgerinnung beeinflussen, Interferonen, Interleukinen, Chemokinen, neutotrophen Faktoren, Proteinen, die das Knochenwachstumbeeinflussen, Hedgehog-Proteinen, Tumorwachstumsfaktoren, Wachstumshormonen, ACTH, Enkephalinen, Endorphinen, Rezeptoren und anderen proteinbindenden Proteinen.

11. Verfahren nach Anspruch 10 zur Herstellung von EPO.

12. Verfahren zur Gewinnung eines Polypeptids,
**dadurch gekennzeichnet,**
**dass** durch ein Verfahren nach einem der Ansprüche 1 bis 7 eine humane Zellinie identifiziert wird, in welcher ein Zielgen, das für ein gewünschtes Polypeptid kodiert, endogen vorliegt, eine Aktivierung des Zielgens erfolgt und dann das von dem Zielgen kodierte Polypeptid gewonnen wird.

13. Verfahren nach Anspruch 12, wobei die Gewinnung des Polypeptids in einem Großfermenter erfolgt.

## Claims

1. Method for selecting human cell lines for the production of human proteins by endogenous activation of a target gene that is present endogenously in the cell line,
**characterized in that**
(a) a human cell line is tested for the presence of the following characteristics:
(i) a target gene with the desired nucleic acid sequence,
(ii) at least 5 population doublings within 14 days in a suspension culture, and
(iii) at least 5 population doublings within 14 days in a serum-free culture medium, and
(b) a cell line fulfilling the characteristics (i), (ii) and (iii) is used as a starting cell line for the endogenous activation of the target gene.

2. Method according to claim 1,
**characterized in that**
a human cell line is used which (iv) has a generation time of 16 to 256 population doublings within one week in a culture medium.

3. Method according to claim 2,
**characterized in that**
a human cell line is used which has 64 to 128 population doublings within one week in a culture medium.

4. Method according to one of the previous claims,
**characterized in that**
a human cell line is used which (v) has essentially no endogenous expression of the target gene.

5. Method according to one of the previous claims,
**characterized in that**
a human cell line is used which (vi) contains more than 2 chromosomal copies of the target gene.

6. Method according to one of the previous claims,
**characterized in that**
a human cell line is used which (vii) synthesizes the cell protein with a glycosylation pattern that is comparable to that of the naturally occurring target protein.

7. Method according to one of the previous claims,
**characterized in that**
a human cell line is used which (viii) is free from detectable infectious contaminations.

8. Method for producing human proteins by endogenous gene activation in a human cell,
**characterized in that**
a cell line which fulfils the characteristics (i), (ii) and (iii) as defined in claim 1 is selected by a method according to one of the claims 1 to 7 and the cell line selected in this manner is used.

9. Method according to claim 8,
**characterized in that**
a cell line is used which additionally fulfils at least one of the characteristics (iv), (v), (vi), (vii) and (viii) as defmed in one of the claims 2, 4, 5, 6 and 7.

10. Method according to claim 8 or 9 for the production of a human factor selected from EPO, TPO, colony-stimulating factors, proteins which affect blood coagulation, interferons, interleukins, chemokines, neurotrophic factors, proteins which affect bone growth, hedgehog proteins, tumour growth factors, growth hormones, ACTH, encephalins, endorphins, receptors and other protein-binding proteins.

11. Method according to claim 10 for the production of EPO.

12. Method for obtaining a polypeptide,
**characterized in that**
a human cell line in which a target gene which codes for a desired polypeptide is present endogenously, is identified by a method according to one of the claims 1 to 7, the target gene is activated and then the polypeptide coded by the target gene is obtained.

13. Method according to claim 12 in which the polypeptide is obtained in a large fermenter.

## Revendications

1. Procédé de sélection de lignées cellulaires humaines pour la production de protéines humaines par l'activation endogène d'un gène cible se trouvant à l'état endogène dans la cellule,
**caractérisé en ce que**
(a) l'on détermine dans une lignée cellulaire humaine, la présence des caractéristiques suivantes :
(i) un gène cible présentant la séquence d'acide nucléique souhaitée,
(ii) au moins 5 doublements de population en l'espace de 14 jours dans une culture en suspension, et
(iii) au moins 5 doublements de population en l'espace de 14 jours dans un milieu de culture dépourvu de sérum, et
(b) on utilise une lignée cellulaire présentant les caractéristiques (i), (ii) et (iii) comme lignée cellulaire de départ pour l'activation endogène du gène cible.

2. Procédé selon la revendication 1, **caractérisé en ce que** l'on utilise une lignée cellulaire humaine (iv) qui présente un temps de génération de 16 à 256 doublements de population en l'espace d'une semaine dans un milieu de culture.

3. Procédé selon la revendication 2, **caractérisé en ce que** l'on utilise une lignée cellulaire humaine qui présente 64 à 128 doublements de population en l'espace d'une semaine dans un milieu de culture.

4. Procédé selon l'une quelconque des revendications précédentes, **caractérisé en ce que** l'on utilise une lignée cellulaire humaine (v) qui ne présente essentiellement pas d'expression endogène du gène cible.

5. Procédé selon l'une quelconque des revendications précédentes, **caractérisé en ce que** l'on utilise une lignée cellulaire humaine (vi) qui contient plus de 2 copies chromosomiques du gène cible.

6. Procédé selon l'une quelconque des revendications précédentes, **caractérisé en ce que** l'on utilise une lignée cellulaire humaine (vii) qui synthétise la protéine cellulaire avec un schéma de glycosylation comparable à celui de la protéine cible présente à l'état naturel.

7. Procédé selon l'une quelconque des revendications précédentes, **caractérisé en ce que** l'on utilise une lignée cellulaire humaine (viii) qui est dépourvue de contaminations infectieuses détectables.

8. Procédé de production de protéines humaines par l'activation d'un gène endogène dans une cellule humaine, **caractérisé en ce qu'**on choisit par un procédé selon l'une quelconque des revendications 1 à 7, une lignée cellulaire qui répond aux caractéristiques (i), (ii) et (iii) telles que définies dans la revendication 1 et que l'on utilise la lignée cellulaire ainsi choisie.

9. Procédé selon la revendication 8, **caractérisé en ce que** l'on utilise une lignée cellulaire qui répond en outre au moins à l'une des caractéristiques (iv), (v), (vi) (vii) et (viii) telles que définies dans l'une des revendications 2, 4, 5, 6 et 7.

10. Procédé selon la revendication 8 ou 9 pour la production d'un facteur humain choisi parmi l'EPO, le TPO, les facteurs de stimulation de colonies, les protéines agissant sur la coagulation sanguine, les interférons, les interleukines, les chémokines, les facteurs neutotrophes, les protéines qui influent sur la croissance osseuse, les protéines Hedgehog, les facteurs de croissance tumorale, les hormones de croissance, l'ACTH, les encéphalines, les endomorphines, les récepteurs et autres protéines de fixation protéique.

11. Procédé selon la revendication 10 pour la fabrication d'EPO.

12. Procédé d'obtention d'un polypeptide, **caractérisé en ce que** l'on identifie par un procédé selon l'une quelconque des revendications 1 à 7, une lignée cellulaire humaine dans laquelle un gène cible qui code pour un polypeptide souhaité, est présent à l'état endogène, l'on effectue une activation du gène cible et ensuite, le polypeptide codé par le gène cible est recueilli.

13. Procédé selon la revendication 12, dans lequel l'obtention du polypeptide s'effectue dans un fermenteur de grande taille.


### SEQUENZPROTOKOLL

(1) ALLGEMEINE ANGABEN:
   (i) ANMELDER:
      (A) NAME: Boehringer Mannheim GmbH
      (B) STRASSE: Sandhofer Str. 112-132
      (C) ORT: Mannheim
      (E) LAND: Deutschland
      (F) POSTLEITZAHL: 68305
   (ii) BEZEICHNUNG DER ERFINDUNG: Identifizierung humaner Zellinien zur Produktion humaner Proteine durch endogene Genaktivierung
   (iii) ANZAHL DER SEQUENZEN: 4
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 32 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 1:
      CGCGGCGGAT CCCAGGGAGC TGGGTTGACC GG 32
(2) ANGABEN ZU SEQ ID NO: 2:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 2:
      GGCCGCGAAT TCTCCGCGCC TGGCCGGGGT CCCTCAGC 38
(2) ANGABEN ZU SEQ ID NO: 3:
   (i) SEQUENZKENNZEICHEN:
      (A) LÄNGE: 36 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 3:
      CGCGGCGGAT CCTCTCCTCC CTCCCAAGCT GCAATC 36
(2) ANGABEN ZU SEQ ID NO: 4:
   (i) SEQUENZKENNZEICHEN:
      (A) LÄNGE: 36 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 4:
      GGCCGCGAAT TCTAGAACAG ATAGCCAGGC TGAGAG 36